# EUROPÄISCHE PATENTSCHRIFT

(11) **EP 2 874 710 B1**
(45) Veröffentlichungstag und Bekanntmachung des Hinweises auf die Patenterteilung: **25.10.2017**
(21) Anmeldenummer: 13727209.2
(22) Anmeldetag: 07.06.2013
(51) Int. Cl.: A61Q 17/04, A61K 8/49, A61K 8/86, A61K 8/04, A61K 8/06

(54) **NEUES KOSMETISCHES SONNENSCHUTZSPRAY**
NEW COSMETIC SUNSCREEN SPRAY
NOUVEAU SPRAY COSMÉTIQUE SOLAIRE

(30) Priorität: 18.07.2012 DE 102012212531
(43) Veröffentlichungstag der Anmeldung: 27.05.2015
(73) Patentinhaber: Beiersdorf AG, 20253 Hamburg (DE)
(72) Erfinder: PETERSEN, Miriam, 22297 Hamburg (DE); KOCH, Petra, 22457 Hamburg (DE); MÖLLGAARD, Svenja Lena, 22337 Hamburg (DE)
(86) Internationale Anmeldenummer: PCT/EP2013/061774
(87) Internationale Veröffentlichungsnummer: WO 2014/012705

(56) Entgegenhaltungen:
- EP-A2- 2 438 903
- WO-A2-2011/141110
- DATABASE GNPD [Online] MINTEL; 1. Juni 2011 (2011-06-01), Energizer Personal Care: "Banana Boat Sin Lagrimas Sunscreen SPF 50", XP002728790, Database accession no. 1556382
- DATABASE GNPD [Online] MINTEL; 1. Juli 2009 (2009-07-01), Deborah Bioetyc Easy Sun: "Sun Lotion High Protection SPF 50", XP002728791, Database accession no. 1146002
- Anonymous: "Gransurf 90", Grant Industries, Inc. , 4. September 2009 (2009-09-04), Seiten 1-2, XP002728792, Gefunden im Internet: URL:https://web.archive.org/web/2009090404 5445/http://www.grantinc.com/cosmetics/spe cialty/surfactants/gransurf_90.php [gefunden am 2014-08-22]

## Beschreibung

Die vorliegende Erfindung betrifft eine kosmetische W/O-Emulsion enthaltend
a) Cetyl PEG/PPG-10/1 Dimethicone sowie
b) 2,4-Bis-{[4-(2-ethyl-hexyloxy)-2-hydroxy]-phenyl}-6-(4-methoxyphenyl)-1,3,5-triazin.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Sonnenschutzmittel werden in der Regel auf der Basis von O/W-Emulsionen (Öl-in-Wasser-Emulsionen) hergestellt, da O/W-Emulsionen aufgrund der äußeren wässrigen Phase eine angenehme Sensorik aufweisen und generell zu höheren Lichtschutzfaktoren führen (siehe Ärztl. Kosmetologie 7, 169-171 (1977)). Darüber hinaus lassen sich, im Gegensatz zur W/O-Emulsion, auf der Basis einer O/W-Emulsion dünnflüssige, gut versprühbare Zubereitungen herstellen. Nachteilig an O/W-Emulsionen ist jedoch der Umstand, dass diese Zubereitungen nicht wasserfest sind. Zur Erhöhung der Wasserfestigkeit müssen diesen Zubereitungen Filmbildner zugesetzt werden, die dann wiederum zu einer klebrig-unattraktiven Sensorik führen.

Es war daher die Aufgabe der vorliegenden Erfindung, ein Sonnenschutzmittel auf der Basis einer W/O-Emulsion zu entwickeln, welches einen hohen Lichtschutzfaktor aufweist und das gleichzeitig dünnflüssig und gut versprühbar ist.

Eine spezielle Form der Sonnenschutzmittel stellen die Sonnensprays dar. Hierbei unterscheidet der Fachmann zwischen sogenannten Pumpsprays, bei denen die Zubereitung mittels einer mechanischen Kolbenpumpe aus dem Vorratsbehältnis gefördert wird, sowie Zubereitungen, die mittels eines Treibgases aus einer Aerosoldose appliziert werden.

Bei diesen Aerosolsprays ist darauf zu achten, dass das Treibgas unter Druck in der Dose flüssig ist und damit nach dem Schütteln der Dose zum homogenen Bestandteil der lipophilen Phase der Emulsion wird. Durch die Lösung des Treibgases in der lipophilen Phase ändert sich deren Polarität und Lösungseigenschaften. Erst nach dem Entweichen der Zubereitung aus der Aerosoldose geht das Treibgas durch den Druckabfall in den gasförmigen Zustand über und entweicht wieder aus der lipophilen Phase. Um eine gute Versprühbarkeit der Zubereitung zu erreichen, muss deshalb dem Umstand Rechnung getragen werden, dass die lipophile Phase mit und ohne Treibgas eine homogene Beschaffenheit behält und es nicht in einem der beiden Zustandsformen (mit und ohne Treibgas) zu einer Ausfällung einzelner Bestandteile der lipophilen Phase, insbesondere von schwer löslichen UV-Filtern, Wachsen und Filmbildnern kommt. Dies ist jedoch bei den Zubereitungen des Standes der Technik regelmäßig der Fall. Dieser Effekt führt dann beispielsweise zur Verstopfung des Sprühkopfes der Aerosoldose.

Es war daher die Aufgabe der vorliegenden Erfindung, ein mit Hilfe eines Treibgases versprühbares Sonnenschutzmittel auf der Basis einer W/O-Emulsion zu entwickeln, bei dem es nicht zu Ausfällungen aus der lipophilen Phase kommt, wenn das Treibgas in der Phase gelöst oder nicht gelöst vorliegt.

Ein weiterer Nachteil an den Zubereitungen des Standes der Technik liegt in der schnellen Entmischung von durch Aufschütteln in der Zubereitung gelöstem Treibgas und Zubereitung. Im Zusammenspiel mit dem Treibgas kommt es bei den Stand der Technik-Rezepturen nach Schütteln zu einer vergleichsweisen schnellen Trennung von Bulk (= Zubereitung) und Gas. Dieser Effekt ist im Glasaerosolgefäß gut zu beobachten und ist für die Anwendung durch den Verbraucher nachteilig. Der Verbraucher muss entweder zeitnah erneut Schütteln oder verwendet ein Gemisch was anteilig zu viel Treibgas bzw. zu wenig Bulk enthält und benutzt unbewusst weniger Sonnenschutzmittel als eigentlich beabsichtigt. Nicht zuletzt führt die Verwendung von zu viel Treibgas dazu, dass sich später nicht mehr die gesamte Zubereitung aus dem Aerosolgefäß entnehmen lässt, da nicht mehr genügend Treibgas zur Entleerung vorhanden ist.

Es war daher die Aufgabe der vorliegenden Erfindung, die Nachteile des Standes der Technik zu beseitigen und ein Aerosol zu entwickeln, bei dem das Treibgas nach dem Schütteln unter Druck über einen längeren Zeitraum in der Zubereitung gelöst verbleibt.

Überraschend gelöst werden die Aufgaben durch eine kosmetische W/O-Emulsion enthaltend
a) Cetyl PEG/PPG-10/1 Dimethicone sowie
b) 2,4-Bis-{[4-(2-ethyl-hexyloxy)-2-hydroxy]-phenyl}-6-(4-methoxyphenyl)-1,3,5-triazin, dadurch gekennzeichnet, dass die Zubereitung mit einem Treibgas versetzt ist und das Treibgas gewählt wird aus Propan, n-Butan, Isobutan und deren Mischungen.

Zwar kennt der Stand der Technik die EP 1500427, EP 2088988, WO 2006/134886, US 7829106, EP 1549281 und DE 102010050774 sowie die EP2438903, WO 2011/141110, die GNPD Datenbankeinträge (Mintel) Nr. 1556382 und 1146002 und die URL:https://web.archive.org/web/2009090404544/http://www.granic.com/cosmetics/speciality /surfactants/grandsurf_90.php vom 4. September 2009, doch konnten diese Schriften nicht den Weg zur vorliegenden Erfindung weisen.

Die Formulierungen "erfindungsgemäße Zubereitung" etc. beziehen sich im Rahmen der vorliegenden Offenbarung auf die erfindungsgemäße W/O-Emulsion, auch wenn dies nicht explizit erwähnt ist.

Alle Konzentrationsangaben im Rahmen der vorliegenden Offenbarung beziehen sich, falls es nicht explizit angegeben ist, auf eine Zubereitung ohne Treibgas.

Die erfindungsgemäßen Zubereitungen sind überraschend temperatur- und lagerstabil und zeichnen sich dabei durch eine überraschend leichte, nicht klebrige Sensorik aus. Neben der klassisch bekannten "Lagerstabilität" bei unterschiedlichen Temperaturen, bezieht sich die Stabilität bei erfindungsgemäßen Zubereitungen zusätzlich auf die langsamere Entmischung nach Schütteln.

Aerosol-Produkte werden vor Anwendung geschüttelt. Nach dem Stand der Technik zeigen die geschüttelten Zubereitungen, bei denen das Treibgas dann in der Ölphase gelöst ist, häufig bei Raumtemperatur eine Ölabscheidung. Erfindungsgemäße Zubereitungen zeigen hingegen deutlich längere Entmischungszeiten nach Schütteln. Dabei kann"länger" in diesem Zusammenhang mehr als 3 Stunden bedeuten. In besonders bevorzugen Ausführungsformen zeigten die Zubereitungen eine Ölabscheidung bei Raumtemperatur von mehr als einem Monat.

Die sensorischen Eigenschaften der erfindungsgemäßen Zubereitungen werden von Anwendern als angenehm und nicht klebrig beurteilt.

Besonders überraschend war nicht zuletzt der Umstand, dass der Lichtschutzfaktor (SPF) der erfindungsgemäßen Zubereitung nach dem Baden ansteigt, wenn der Anwender die erfindungsgemäße Zubereitung auf die Haut aufgetragen hat und anschließend mit Wasser in Kontakt kommt.

Erfindungsgemäß bevorzugte Ausführungsformen der vorliegenden Erfindung sind dadurch gekennzeichnet, dass die Zubereitung 2,4-Bis-{[4-(2-ethyl-hexyloxy)-2-hydroxy]-phenyl}-6-(4-methoxyphenyl)-1,3,5-triazin in einer Konzentration von 0,5 bis 10 Gewichts-%, bezogen auf das Gesamtgewicht der Zubereitung, enthält.

Außerdem ist es erfindungsgemäß bevorzugt, wenn die Zubereitung Cetyl PEG/PPG-10/1 Dimethicone in einer Konzentration von 0,5 bis 3 Gewichts-%, bezogen auf das Gesamtgewicht der Zubereitung, enthält.

Dabei ist es erfindungsgemäß besonders bevorzugt, wenn die Zubereitung Cetyl PEG/PPG-10/1 Dimethicone in einer Konzentration von 1,5 bis 2 Gewichts-% und ganzbesonders bevorzugt 1,7 Gewichts-%, jeweils bezogen auf das Gesamtgewicht der Zubereitung, enthält.

Alle Konzentrationsangaben im Rahmen der vorliegenden Offenbarung beziehen sich, falls es nicht explizit angegeben ist, auf eine Zubereitung ohne Treibgas.

Es ist erfindungsgemäß vorteilhaft, wenn die erfindungsgemäße Zubereitung einen oder mehrere UV-Filter enthält, gewählt aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornyliden-methyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 4-(tert.-Butyl)-4'-methoxydibenzoylmethan; 2-Ethylhexyl-2-cyano-3,3-diphenylacrylat; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Titandioxid; Zinkoxid.

Erfindungsgemäß vorteilhaft enthält die erfindungsgemäße Zubereitung UV-Filter in einer Menge von 1 bis 30 Gewichts-%, bezogen auf das Gesamtgewicht der Zubereitung.

Erfindungsgemäß bevorzugt ist die erfindungsgemäße Zubereitung frei ist von 3-(4-Methylbenzyliden)campher.

Erfindungsgemäß bevorzugt ist die erfindungsgemäße Zubereitung frei ist von Benzophenone-3 bzw. Benzophenone-4.

Es ist erfindungsgemäß vorteilhaft, wenn die Zubereitung 4-(tert.-Butyl)-4'-methoxydiben-zoylmethan und/oder 2-(4'-Diethylamino-2'-hydoxybenzoyl)-benzoesäurehexylester enthält.

Erfindungsgemäß besonders bevorzugt ist dabei ein Gehalt an 4-(tert.-Butyl)-4'-methoxydi-benzoylmethan.

4-(tert.-Butyl)-4'-methoxydibenzoylmethan und 2-(4'-Diethylamino-2'-hydoxybenzoyl)-benzoesäurehexylester werden dabei erfindungsgemäß vorteilhaft einzeln oder als Mischung (bevorzugt einzeln) in einer Gesamtkonzentration von 0,5 bis 10 Gewichts-%, bezogen auf das Gesamtgewicht der Zubereitung eingesetzt. Erfindungsgemäß bevorzugt ist ein Gehalt von 3,5 bis 5 Gewichts-% und besonders bevorzugt von 4,5 Gewichts-%, jeweils bezogen auf das Gesamtgewicht der Zubereitung, eingesetzt.

Es ist erfindungsgemäß besonders bevorzugt, wenn die Zubereitung Octocrylen enthält.

Enthält die Zubereitung Octocrylen, so wird diese Verbindung erfindungsgemäß vorteilhaft in einer Konzentration von 0,5 bis 10 Gewichts-%, bezogen auf das Gesamtgewicht der Zubereitung eingesetzt.

Es ist erfindungsgemäß vorteilhaft, wenn die Zubereitung Polyglyceryl-4 Diisostearate/Polyhydroxystearate/Sebacate und/oder Diisostearoyl Polyglyceryl-3 Dimer Dioleate enthält.

Es ist erfindungsgemäß, wenn die erfindungsgemäße Zubereitung mit einem Treibgas versetzt wird. Dies geschieht erfindungsgemäß bevorzugt in einer Aerosoldose. Mit diesem Treibgas kann die Zubereitung dann erfindungsgemäß vorteilhaft versprüht werden. Diese Zubereitungen weisen ein überraschend leichtes sensorisches Gefühl auf der Haut auf.

Erfindungsgemäß besonders bevorzugt, ist eine Mischung des Treibgases aus 60 Gewichts-% n-Butan, 20 Gewichts-% Isobutan und 20 Gewichts-% Propan (Druckstufe 2,7 bar) oder 72 Gewichts-% Isobutan +23 Gewichts-% Propan + 5 Gewichts-% n-Butan (Druckstufe 3,5 bar), wobei Schwankungen von jeweils 10% noch als erfindungsgmäß gelten.

Wird die erfindungsgsgemäße Zubereitung mit Treibgas versetzt, so ist es erfindungsgemäß vorteilhaft, wenn das Verhältnis von Zubereitung zu Treibgas von 60-80 Gewichts-% Zubereitung zu 40-20 Gewichts-% Treibgas.beträgt.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile® (CAS-Nr. 7631-86-9), Talkum, Lauroyl Lysine und Acrylonitrile-methacrylonitrile-methyl-methacrylate.

Erfindungsgemäß bevorzugt ist es, wenn die Zubereitung Füllstoffe, insbesondere Polyethylen, Nylon, natürliche oder modifizierte Stärken wie Tapiokastärke und/oder Silikate wie beispielsweise Talkum enthält.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl wie Isopropanol, Diole oder Polyole niedriger C-Zahl sowie deren Ether, vorzugsweise Propylenglykol, 2-Methylpropan-1,3-diol, Pentan-1,2-diol, Hexan-1,2-diol, Octan-1,2-diol, Decan-1,2-diol, Glycerin, Ethylenglykol, Ethylenglykolmonoethyl-oder-monobutylether, Propylenglykolmonomethyl, -monoethyl- oder -monobutylether, Diethylenglykolmonomethyl- oder -monoethylether und analoge Produkte, Schaumstabilisatoren, Elektrolyte, etc.. Die erfindungsgemäße Zubereitung kann vorteilhaft Konsistenzbildner (Gelbildner, Verdickungsmittel) wie beispielsweise Polyacrylate (auch quervernetzt) oder Cellulosederivate (beispielsweise Hydroxyethylcellulose) oder andere enthalten.

Erfindungsgemäß bevorzugt ist es, wenn die erfindungsgemäße Zubereitung dadurch gekennzeichnet ist, dass die Zubereitung Propylenglycol, Butylenglycol, 2-Methylpropan-1,3-diol, 1,2-Pentandiol, 1,2-Hexandiol, 1,2-Octandiol und/oder 1,2-Decandiol enthält. Dabei werden diese Diole/Glycole erfindungsgemäß vorteilhaft in einer Gesamtmenge von 0,1 bis 3 Gewichts-%, bezogen auf das Gesamtgewicht der Zubereitung, eingesetzt.

Es ist erfindungsgemäß bevorzugt, wenn die Zubereitung Xanthangummi enthält.

Es ist erfindungsgemäß vorteilhaft, wenn die Zubereitung mit Magnesiumsulfat oder Natriumchlorid in einer Konzentration von 0,1 bis 3 Gewichts-%, bezogen auf das Gesamtgewicht der Zubereitung stabilisiert ist. Dabei ist der Einsatz von Magnesiumsulfat erfindungsgemäß bevorzugt.

Selbstverständlich kann die Zubereitung mit den üblichen, in der Kosmetik verwendeten Konservierungsmitteln konserviert werden.

Dabei ist es erfindungsgemäß bevorzugt, wenn die Zubereitung Phenoxyethanol enthält.

Darüber hinaus ist es erfindungsgemäß vorteilhaft, wenn die erfindungsgemäße Zubereitung Kaliumsorbat enthält, das erfindungsgemäß bevorzugt in einer Konzentration von 0,05 bis 0,2 Gewichts-%, bezogen auf das Gesamtgewicht der Zubereitung, eingesetzt wird.

Darüber hinaus ist es erfindungsgemäß bevorzugt, wenn die Zubereitung frei ist von Parabenen.

Die Ölphase der erfindungsgemäßen Zubereitung wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Jojobaöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Phenethylbenzoat, 2-Phenylethylbenzoat, Isopropyl Lauroyl Sarkosinat, Phenyl Trimethicon, Cyclomethicon, Dibutyladipat, Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol* CC bei der Fa. Cognis erhältliche.

Es ist ferner vorteilhaft, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Tridecylsalicylat (welches unter der Handelsbezeichnung Cosmacol ESI bei der Fa. Sasol erhältlich ist), C12-C15 Alkylsalicylat (unter der Handelsbezeichnung Dermol NS bei der Fa. Alzo erhältlich), Hexadecylbenzoat und Butyloctylbenzoat und Gemische davon (*Hallstar AB*).

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene, C13-16 Isoparaffin und Isohexadecan. Unter den Polyolefinen sind Polydecene die bevorzugten Substanzen.

Es ist erfindungsgemäß vorteilhaft, wenn die erfindungsgemäße Zubereitung einen oder mehrere Wirkstoffe gewählt aus der Gruppe der Verbindungen Gylcyrrhetinsäure, Harnstoff, Arctiin, alpha-Liponsäure, Folsäure, Phytoen, D-Biotin, Coenzym Q10, alpha-Glucosylrutin, Carnitin, Carnosin, Coffein, natürliche und/oder synthetische Isoflavonoide, Glycerylglucose, Kreatin, Kreatinin, Taurin, Tocopherol, Tocopherolacetat, β-Alanin und/oder Licochalcon A, enthält.

Erfindungsgemäß vorteilhaft wird die erfindungsgemäße Zubereitung in einer Treibgasdose enthaltend die kosmetische Zubereitung, dargereicht.

Dabei ist es erfindungsgemäß bevorzugt, wenn die Dose eine Aluminiumdose ist, die auf der Innenseite mit einem Schutzlack beschichtet ist.

In einem solchen Falle ist es erfindungsgemäß vorteilhaft, wenn der Schutzlack ein Polyamid-Imid-Lack oder ein Pulverlack ist.

Erfindungsgemäß vorteilhafte Polyamid-Imid-(PAM)- Lacke sind beispielsweise PPG8460-303A Gold PAM-2-C Internal Liner 8460N von der Firma HOBA.

Ein erfindungsgemäß vorteilhafter Pulverlack ist beispielsweise der Epoxy Pulverlack Drylac 069/10103 von der Firma Tiger.

Es ist erfindungsgemäß vorteilhaft, wenn das Ventil der Treibgasdose im Sprühkopf ein Ariane Kugelventil ist. Ein Beispiel hierfür sind Kugelventile der Firma Aptar.

Das Ventil hat erfindungsgemäß vorteilhaft einen Aluteller, der lackiert ist mit z.B. Micoflex-Lack (L6X392L/5 basecoat mit L3E692S/5 topcoat oder L6X392L/6 basecoat mit L3E692S/6 topcoat) von Valspar oder Heliotherm Schutzlack VP 16506.

Die Kegelbohrung kann erfindungsgemäß vorteilhaft 1x 0,32 mm oder 1x 0,40mm oder 1x 0,5mm betragen. Mögliche Tellerdichtungen sind z.B. Nitril B 175, Nitril BA 7402, Nitril KA 6712, Nitril Buna 1602, Chlorbutyl CA 6600, Neoprene NA 7202, Butyl U 133, Buna 5252 6.

Darüber hinaus sind solche Treibbgasdosen erfindungsgemäß vorteilhaft dadurch gekennzeichnet, dass als Sprühkopf z.B. der WS 25 von Aptar eingesetzt wird, mit folgenden Düsen: WAZ 4833, WAZ 5118, WAZ 3832 oder WAZ AT 5034.

Die erfindungsgemäßen Zubereitungen werden erfindungsgemäß bevorzugt als Sonnenschutzmittel verwendet. Darüber hinaus ist ihr Einsatz als Tagespflegeprodukt erfindungsgemäß.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

Die Zubereitungen (=Bulk) wurden in eine Aerosoldose abgefüllt, die auf der Innenseite mit einem Polyamid-Imid-Lack (PPG8460-303A Gold PAM-2-C Internal Liner 8460N von der Firma HOBA) oder mit dem Epoxy Pulverlack Drylac 069/10103 von der Firma Tiger beschichtet war und mit der Treibgasmischung versetzt.

Als Sprühkopf wurde Sprühkopf WS 25 von Aptar eingesetzt wird, mit folgenden Düsen: WAZ 4833, WAZ 5118, WAZ 3832 oder WAZ AT 5034.

Als Ventil der Treibgasdose im Sprühkopf wurde ein Ariane Kugelventil ist der Firma Aptar verwendet, welches einen lackierten Aluminiumteller aufwies. Dabei wurden Kegelbohrungen von 1x 0,32 mm oder 1x 0,40mm oder 1x 0,5mm verwendet.

| **INCI** | **1** | **2** | **3** | **4** | **5** |
|---|---|---|---|---|---|
| C13-16 Isoparaffin | 12 | 12 | 0 | | |
| Butylene Glycol Dicaprylate/Dicaprate | 8,5 | 8,5 | 8,5 | 14 | 14 |
| Isohexadecane | | | 12 | | |
| Isopropyl Stearate | | | | 12 | 12 |
| Cetyl PEG/PPG-10/1 Dimethicone | 1,7 | 1,7 | 1,7 | 1,7 | 2 |
| Parfum | 0,3 | 0,3 | 0,3 | 0,3 | 0,3 |
| Glycerin | 4,95 | 4,95 | 4,95 | 4,95 | 4,95 |
| Butylene Glycol | | | | 5 | |
| Sodium Hydroxide | | | 0,225 | | |
| Potassium Sorbate | 0,125 | | | | |
| Ethylparaben | | 0,1 | 0,1 | 0,1 | 0,1 |
| Methylparaben | | 0,3 | 0,3 | 0,3 | 0,3 |
| Phenoxyethanol | | 0,5 | 0,5 | 0,5 | 0,5 |
| Sodium Chloride | | 0 | | 3 | 1,5 |
| Aqua | ad 100 | ad 100 | ad 100 | ad 100 | ad 100 |
| Trisodium EDTA | 0,2 | 0,2 | 0,2 | 0,2 | 0,2 |
| Magnesium Sulfate | 0,7 | 0,7 | 0,7 | | |
| Homosalate | 9,5 | 9,5 | | 9,5 | 9,5 |
| Octocrylene | 9,5 | 9,5 | 7 | 9,5 | 9,5 |
| Ethylhexyl Salicylate | 4,5 | 4,5 | 4,5 | 4,5 | 4,5 |
| Bis-Ethylhexyloxyphenol Methoxyphenyl Triazine | 2 | 2 | 2,5 | 2 | 2 |
| Butyl Methoxydibenzoylmethane | 4,5 | 4,5 | 4,5 | 4,5 | 4,5 |
| Phenylbenzimidazole Sulfonic Acid | | | 1,5 | | |

Der Ansatz setzt sich zusammen aus 75% Bulk und 25 % Treibgas (60% Butane + 20% Isobutane + 20% Propane, Druckstufe 2,7 bar)

| **INCI** | **6** | **7** | **8** | **9** | **10** |
|---|---|---|---|---|---|
| Piroctone Olamine | 0,05 | | 0,05 | 0,05 | 0,05 |
| C13-16 Isoparaffin | 12 | 12 | 12 | 12 | 10,5 |
| Butylene Glycol Dicaprylate/Dicaprate | 8,5 | 8,5 | 8,5 | 8,5 | 10 |
| Cetyl PEG/PPG-10/1 Dimethicone | 1,7 | 1,7 | 1,7 | 1,7 | 1,7 |
| Parfum | 0,3 | 0,3 | 0,3 | 0,3 | 0,3 |
| Glycerin | 4,95 | 4,95 | 4,95 | 4,95 | 4,95 |
| Sodium Hydroxide | 0,225 | 0,225 | 0,216 | 0,216 | 0,216 |
| Phenoxyethanol | 0,5 | 0,5 | 0,5 | 0,5 | 0,5 |
| Ethylparaben | | 0,1 | | | |
| Methylparaben | | 0,3 | | | |
| Sodium Chloride | | 0 | 1,5 | | 3 |
| Potassium Chloride | | 0 | | 3 | |
| Aqua | ad 100 | ad 100 | ad 100 | ad 100 | ad 100 |
| Trisodium EDTA | 0,2 | 0,2 | 0,2 | 0,2 | 0,2 |
| Magnesium Sulfate | 0,7 | 0,7 | | | |
| Octocrylene | 7 | 7 | 7 | 7 | 7 |
| Ethylhexyl Salicylate | 4,5 | 4,5 | 4,5 | 4,5 | 4,5 |
| Bis-Ethylhexyloxyphenol Methoxyphenyl Triazine | 2,5 | 2,5 | 2,5 | 2,5 | 2,5 |
| Butyl Methoxydibenzoylmethane | 4,5 | 4,5 | 4,5 | 4,5 | 4,5 |
| Phenylbenzimidazole Sulfonic Acid | 1,5 | 1,5 | 1,5 | 1,5 | 1,5 |

Der Ansatz setzt sich zusammen aus 75% Bulk und 25% Treibgas (72% Isobutan +23% Propan + 5% n-Butan, Druckstufe 3,5 bar)

| **INCI** | **11** | **12** | **13** | **14** |
|---|---|---|---|---|
| Piroctone Olamine | | 0,05 | 0,05 | |
| C13-16 Isoparaffin | 12 | 12 | 14 | 10,5 |
| Butylene Glycol Dicaprylate/Dicaprate | 8,5 | 14 | 14 | 10 |
| Cetyl PEG/PPG-10/1 Dimethicone | 1,7 | 1,7 | 1,7 | 2,5 |
| Polyglyceryl-4 Diisostearate/Polyhydroxystearate/Sebacate | 3 | | | |
| Parfum | 0,3 | 0,3 | 0,3 | 0,3 |
| Glycerin | 4,95 | 4,95 | 4,95 | 4,95 |
| Sodium Hydroxide | | 0,216 | 0,216 | 0,216 |
| Ethylparaben | 0,1 | | | 0,1 |
| Methylparaben | 0,3 | | | 0,3 |
| Phenoxyethanol | 0,5 | 0,5 | 0,5 | 0,5 |
| Sodium Chloride | | 3 | 3 | 3 |
| Aqua | ad 100 | ad 100 | ad 100 | ad 100 |
| Trisodium EDTA | 0,2 | 0,2 | 0,2 | 0,2 |
| Magnesium Sulfate | 0,7 | | | |
| Homosalate | 9 | | | |
| Octocrylene | 9 | 7 | 7 | 7 |
| Ethylhexyl Salicylate | 4,5 | 4,5 | 4,5 | 4,5 |
| Bis-Ethylhexyloxyphenol Methoxyphenyl Triazine | 2 | 2,5 | 2,5 | 2,5 |
| Polysilicone-15 | 1 | | | |
| Butyl Methoxydibenzoylmethane | 4,5 | 4,5 | 4,5 | 4,5 |
| Phenylbenzimidazole Sulfonic Acid | | 1,5 | 1,5 | 1,5 |

Der Ansatz setzt sich zusammen aus 75% Bulk und 25 % Treibgas (60% Butane + 20% Isobutane + 20% Propane, Druckstufe 2,7 bar)

## Patentansprüche

1. Kosmetische W/O-Emulsion enthaltend
a) Cetyl PEG/PPG-10/1 Dimethicone sowie
b) 2,4-Bis-{[4-(2-ethyl-hexyloxy)-2-hydroxy]-phenyl}-6-(4-methoxyphenyl)-1,3,5-triazin, **dadurch gekennzeichnet, dass** die Zubereitung mit einem Treibgas versetzt ist und das Treibgas gewählt wird aus Propan, n-Butan, Isobutan und deren Mischungen.

2. Kosmetische Zubereitung nach Anspruch 1, **dadurch gekennzeichnet, dass** die Zubereitung einen oder mehrere UV-Filter enthält, gewählt aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]di-siloxanyl]propyl]-phenol; 3-(4-Methylbenzyliden)campher; 3-Benzylidencampher; Ethylhexylsalicylat; Terephthalidendicamphersulfonsäure; 4-(tert.-Butyl)-4'-methoxydi-benzoylmethan; 2-Ethylhexyl-2-cyano-3,3-diphenylacrylat; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoësäure-tris-(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Titandioxid; Zinkoxid.

3. Kosmetische Zubereitung nach einem der vorhergehenden Ansprüche, **dadurch gekennzeichnet, dass** die Zubereitung 4-(tert.-Butyl)-4'-methoxydibenzoylmethan und/oder 2-(4'-Diethylamino-2'-hydoxybenzoyl)-benzoesäurehexylester enthält.

4. Kosmetische Zubereitung nach einem der vorhergehenden Ansprüche, **dadurch gekennzeichnet, dass** die Zubereitung 2,4-Bis-{[4-(2-ethyl-hexyloxy)-2-hydroxy]-phenyl}-6-(4-methoxyphenyl)-1,3,5-triazin in einer Konzentration von 0,5 bis 10 Gewichts-%, bezogen auf das Gesamtgewicht der Zubereitung, enthält.

5. Kosmetische Zubereitung nach einem der vorhergehenden Ansprüche, **dadurch gekennzeichnet, dass** die Zubereitung Octocrylen enthält.

6. Kosmetische Zubereitung nach einem der vorhergehenden Ansprüchen, **dadurch gekennzeichnet, dass** die Zubereitung Cetyl PEG/PPG-10/1 Dimethicone in einer Konzentration von0,5 bis 3 Gewichts-%, bezogen auf das Gesamtgewicht der Zubereitung, enthält.

7. Kosmetische Zubereitung nach einem der vorhergehenden Ansprüche, **dadurch gekennzeichnet, dass** die Zubereitung einen oder mehrere Wirkstoffe gewählt aus der Gruppe der Verbindungen Gylcyrrhetinsäure, Harnstoff, Arctiin, alpha-Liponsäure, Folsäure, Phytoen, D-Biotin, Coenzym Q10, alpha-Glucosylrutin, Carnitin, Carnosin, Coffein, natürliche und/oder synthetische Isoflavonoide, Glycerylglucose, Kreatin, Kreatinin, Taurin, Tocopherol, Tocopherolacetat, β-Alanin und/oder Licochalcon A, enthält.

8. Kosmetische Zubereitung nach einem der vorhergehenden Ansprüche, **dadurch gekennzeichnet, dass** die Zubereitung Propylenglycol, Butylenglycol, 2-Methylpropan-1,3-diol, 1,2-Pentandiol, 1,2-Hexandiol, 1,2-Octandiol und/oder 1,2-Decandiol enthält.

9. Kosmetische Zubereitung nach einem der vorhergehenden Ansprüche, **dadurch gekennzeichnet, dass** die Zubereitung Phenoxyethanol enthält.

10. Kosmetische Zubereitung nach einem der vorhergehenden Ansprüche, **dadurch gekennzeichnet, dass** die Zubereitung Xanthangummi enthält.

11. Kosmetische Zubereitung nach einem der vorhergehenden Ansprüche, **dadurch gekennzeichnet, dass** die Zubereitung Füllstoffe, insbesondere Polyethylen, Nylon, natürliche oder modifizierte Stärken wie Tapiokastärke und/oder Silikate wie beispielsweise Talkum enthält.

12. Kosmetische Zubereitung nach einem der vorhergehenden Ansprüche, **dadurch gekennzeichnet, dass** die Zubereitung frei ist von Parabenen.

13. Kosmetische Zubereitung nach einem der vorhergehenden Ansprüchen, **dadurch gekennzeichnet, dass** die Zubereitung frei ist von 3-(4-Methylbenzyliden)campher.

14. Treibgasdose enthaltend ein Aerosolspray nach einem der vorhergehenden Ansprüchen, **dadurch gekennzeichnet, dass** die Dose eine Aluminiumdose ist, die auf der Innenseite mit einem Schutzlack beschichtet ist.

15. Treibgasdose nach Anspruch 14, **dadurch gekennzeichnet, dass** der Schutzlack ein Polyamid-Imid-Lack oder ein Pulverlack ist.

16. Treibgasdose nach Anspruch 14 oder 15, **dadurch gekennzeichnet, dass** das Ventil im Sprühkopf ein Ariane Kugelventil ist.

## Claims

1. Cosmetic W/O emulsion comprising
a) cetyl PEG/PPG-10/1 dimethicone and
b) 2,4-bis{[4-(2-ethylhexyloxy)-2-hydroxy]phenyl}-6-(4-methoxyphenyl)-1,3,5-triazine, **characterized in that** the preparation is transferred with a propellant and the propellant is selected from propane, n-butane, isobutane and mixtures thereof.

2. Cosmetic preparation according to Claim 1, **characterized in that** the preparation comprises one or more UV filters selected from the group of compounds comprising 2-phenylbenzimidazole-5-sulfonic acid and/or salts thereof; phenylene-1,4-bis(2-benzimidazyl)-3,3'-5,5'-tetrasulfonic acid salts; 1,4-di(2-oxo-10-sulpho-3-bornylidenemethyl)benzene and salts thereof; 4-(2-oxo-3-bornylidenemethyl)benzenesulfonic acid salts; 2-methyl-5-(2-oxo-3-bornylidenemethyl)sulfonic acid salts; 2,2'-methylenebis(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]disiloxanyl]propyl]phenol; 3-(4-methylbenzylidene)camphor; 3-benzylidenecamphor; ethylhexyl salicylate; terephthalidenedicamphorsulfonic acid; 4-(tert-butyl)-4'-methoxydibenzoylmethane; 2-ethylhexyl 2-cyano-3,3-diphenylacrylate; 2-ethylhexyl 4-(dimethylamino)benzoate; amyl 4-(dimethylamino)benzoate; di(2-ethylhexyl) 4-methoxybenzalmalonate; 2-ethylhexyl 4-methoxycinnamate; isoamyl 4-methoxycinnamate; 2-hydroxy-4-methoxybenzophenone, 2-hydroxy-4-methoxy-4'-methylbenzophenone; 2,2'-dihydroxy-4-methoxybenzophenone; hexyl 2-(4'-diethylamino-2'-hydroxybenzoyl)benzoate; homomenthyl salicylate; 2-ethylhexyl 2-hydroxybenzoate; dimethicodiethylbenzalmalonate; 3-(4-(2,2-bis ethoxycarbonylvinyl)phenoxy)propenyl)methoxysiloxane / dimethylsiloxane - copolymer; dioctylbutylamidotriazone (INCI: Diethylhexyl Butamidotriazone); 2,4-bis[5-1(dimethylpropyl)benzoxazol-2-yl-(4-phenyl)imino]-6-(2-ethylhexyl)imino-1,3,5-triazine with (CAS No. 288254-16-0); tris(2-ethylhexyl) 4,4',4"-(1,3,5-triazine-2,4,6-triyltriimino)trisbenzoate (also: 2,4,6-tris[anilino-(p-carbo-2'-ethyl-1'-hexyloxy)]-1,3,5-triazine (INCI: Ethylhexyl Triazone); 2,4,6-tribiphenyl-4-yl-1,3,5-triazine; merocyanine; titanium dioxide; zinc oxide.

3. Cosmetic preparation according to either of the preceding claims, **characterized in that** the preparation comprises 4-(tert-butyl)-4'-methoxydibenzoylmethane and/or hexyl 2-(4'-diethylamino-2'-hydroxybenzoyl)benzoate.

4. Cosmetic preparation according to any of the preceding claims, **characterized in that** the preparation comprises 2,4-bis{[4-(2-ethylhexyloxy)-2-hydroxy]phenyl}-6-(4-methoxyphenyl)-1,3,5-triazine at a concentration of 0.5 to 10% by weight, based on the total weight of the preparation.

5. Cosmetic preparation according to any of the preceding claims, **characterized in that** the preparation comprises octocrylene.

6. Cosmetic preparation according to any of the preceding claims, **characterized in that** the preparation comprises cetyl PEG/PPG-10/1 dimethicone at a concentration of 0.5 to 3% by weight, based on the total weight of the preparation.

7. Cosmetic preparation according to any of the preceding claims, **characterized in that** the preparation comprises one or more active ingredients selected from the group of compounds comprising glycyrrhetic acid, urea, arctiin, alpha-lipoic acid, folic acid, phytoene, D-biotin, coenzyme Q10, alpha-glucosylrutin, carnitine, carnosine, caffeine, natural and/or synthetic isoflavonoids, glycerylglucose, creatine, creatinine, taurine, tocopherol, tocopherol acetate, β-alanine and/or licochalcone A.

8. Cosmetic preparation according to any of the preceding claims, **characterized in that** the preparation comprises propylene glycol, butylene glycol, 2-methylpropane-1,3-diol, 1,2-pentanediol, 1,2-hexanediol, 1,2-octanediol and/or 1,2-decanediol.

9. Cosmetic preparation according to any of the preceding claims, **characterized in that** the preparation comprises phenoxyethanol.

10. Cosmetic preparation according to any of the preceding claims, **characterized in that** the preparation comprises xanthan gum.

11. Cosmetic preparation according to any of the preceding claims, **characterized in that** the preparation comprises fillers, in particular polyethylene, nylon, natural or modified starches such as tapioca starch and/or silicates such as talc.

12. Cosmetic preparation according to any of the preceding claims, **characterized in that** the preparation is free of parabens.

13. Cosmetic preparation according to any of the preceding claims, **characterized in that** the preparation is free of 3-(4-methylbenzylidene)camphor.

14. Propellant can comprising an aerosol spray according to any of the preceding claims, **characterized in that** the can is an aluminium can which is coated on the inside with a protective coating.

15. Propellant can according to Claim 14, **characterized in that** the protective coating is a polyamide-imide coating or a powder coating.

16. Propellant can according to Claim 14 or 15, **characterized in that** the valve in the spray head is an Ariane ball valve.

## Revendications

1. Émulsion cosmétique E/H contenant :
a) de la cétyl PEG/PPG-10/1 diméthicone, et
b) de la 2,4-bis-{[4-(2-éthyl-hexyloxy)-2-hydroxy]-phényl}-6-(4-méthoxyphényl)-1,3,5-triazine,
**caractérisée en ce que** la préparation est mélangée avec un gaz propulseur et le gaz propulseur est choisi parmi le propane, le n-butane, l'isobutane et leurs mélanges.

2. Préparation cosmétique selon la revendication 1, **caractérisée en ce que** la préparation contient un ou plusieurs filtres UV, choisis dans le groupe constitué par les composés acide 2-phénylbenzimidazole-5-sulfonique et/ou ses sels ; sels de l'acide phénylène-1,4-bis-(2-benzimidazyl)-3,3'-5,5'-tétrasulfonique ; 1,4-di(2-oxo-10-sulfo-3-bornylidène-méthyl)-benzène et ses sels ; sels de l'acide 4-(2-oxo-3-bornylidène-méthyl)benzène-sulfonique ; sels de l'acide 2-méthyl-5-(2-oxo-3-bornylidène-méthyl)sulfonique ; 2,2'-méthylène-bis-(6-(2H-benzotriazol-2-yl)-4-(1,1,3,3-tétraméthylbutyl)-phénol) ; 2-(2H-benzotriazol-2-yl)-4-méthyl-6-[2-méthyl-3-[1,3,3,3-tétraméthyl-1-[(triméthylsilyl)oxy]disiloxanyl]propyl]-phénol ; 3-(4-méthylbenzylidène)camphre ; 3-benzylidène-camphre ; salicylate d'éthylhexyle ; acide téréphtalidène-dicamphre-sulfonique ; 4-(tert.-butyl)-4'-méthoxydibenzoylméthane ; acrylate de 2-éthylhexyl-2-cyano-3,3-diphényle ; ester 2-éthylhexylique de l'acide 4-(diméthylamino)-benzoïque ; ester amylique de l'acide 4-(diméthylamino)benzoïque ; ester di(2-éthylhexylique) de l'acide 4-méthoxybenzalmalonique ; ester 2-éthylhexylique de l'acide 4-méthoxycinnamique ; ester isoamylique de l'acide 4-méthoxycinnamique ; 2-hydroxy-4-méthoxybenzophénone, 2-hydroxy-4-méthoxy-4'-méthylbenzophénone ; 2,2'-dihydroxy-4-méthoxybenzophénone ; ester hexylique de l'acide 2-(4'-diéthylamino-2'-hydroxybenzoyl)-benzoïque ; salicylate d'homomenthyle ; 2-hydroxybenzoate de 2-éthylhexyle ; benzalmalonate de diméthicodiéthyle ; copolymère de 3-(4-(2,2-bis-éthoxycarbonylvinyl)-phénoxy)propényl)-méthoxysiloxane/diméthylsiloxane ; dioctylbutylamidotriazone (INCI : Diethylhexyl-Butamidotriazone) ; 2,4-bis-[5-1(diméthyl-propyl)benzoxazol-2-yl-(4-phényl)-imino]-6-(2-éthylhexyl)-imino-1,3,5-triazine de n° CAS 288254-16-0 ; ester tris-(2-éthylhexylique) de l'acide 4,4',4"-(1,3,5-triazine-2,4,6-triyltriimino)-tris-benzoïque (également : 2,4,6-tris-[anilino-(p-carbo-2'-éthyl-1'-hexyloxy)]-1,3,5-triazine (INCI : Ethylhexyl Triazone) ; 2,4,6-tribiphényl-4-yl-1,3,5-triazine ; mérocyanine ; dioxyde de titane ; oxyde de zinc.

3. Préparation cosmétique selon l'une quelconque des revendications précédentes, **caractérisée en ce que** la préparation contient du 4-(tert.-butyl)-4'-méthoxydibenzoylméthane et/ou de l'ester hexylique de l'acide 2-(4'-diéthylamino-2'-hydroxybenzoyl)-benzoïque.

4. Préparation cosmétique selon l'une quelconque des revendications précédentes, **caractérisée en ce que** la préparation contient de la 2,4-bis-{[4-(2-éthyl-hexyloxy)-2-hydroxy]-phényl}-6-(4-méthoxyphényl)-1,3,5-triazine en une concentration de 0,5 à 10 % en poids, par rapport au poids total de la préparation.

5. Préparation cosmétique selon l'une quelconque des revendications précédentes, **caractérisée en ce que** la préparation contient de l'octocrylène.

6. Préparation cosmétique selon l'une quelconque des revendications précédentes, **caractérisée en ce que** la préparation contient de la cétyl PEG/PPG-10/1 diméthicone en une concentration de 0,5 à 3 % en poids, par rapport au poids total de la préparation.

7. Préparation cosmétique selon l'une quelconque des revendications précédentes, **caractérisée en ce que** la préparation contient un ou plusieurs agents actifs choisis dans le groupe constitué par les composés acide glycyrrhizique, urée, arctiine, acide alpha-liponique, acide folique, phytoène, D-biotine, coenzyme Q10, alpha-glucosylrutine, carnitine, carnosine, caféine, isoflavonoïdes naturels et/ou synthétiques, glycérylglucose, créatine, créatinine, taurine, tocophérol, acétate de tocophérol, β-alanine et/ou licochalcone A.

8. Préparation cosmétique selon l'une quelconque des revendications précédentes, **caractérisée en ce que** la préparation contient du propylène glycol, du butylène glycol, du 2-méthylpropane-1,3-diol, du 1,2-pentanediol, du 1,2-hexanediol, du 1,2-octanediol et/ou du 1,2-décanediol.

9. Préparation cosmétique selon l'une quelconque des revendications précédentes, **caractérisée en ce que** la préparation contient du phénoxyéthanol.

10. Préparation cosmétique selon l'une quelconque des revendications précédentes, **caractérisée en ce que** la préparation contient de la gomme xanthane.

11. Préparation cosmétique selon l'une quelconque des revendications précédentes, **caractérisée en ce que** la préparation contient des charges, notamment du polyéthylène, du nylon, des amidons naturels ou modifiés tels que l'amidon de tapioca et/ou des silicates tels que par exemple le talc.

12. Préparation cosmétique selon l'une quelconque des revendications précédentes, **caractérisée en ce que** la préparation est exempte de parabènes.

13. Préparation cosmétique selon l'une quelconque des revendications précédentes, **caractérisée en ce que** la préparation est exempte de 3-(4-méthylbenzylidène)camphre.

14. Boîte de gaz propulseur contenant un spray aérosol selon l'une quelconque des revendications précédentes, **caractérisée en ce que** la boîte est une boîte en aluminium qui est revêtue sur le côté intérieur avec un vernis protecteur.

15. Boîte de gaz propulseur selon la revendication 14, **caractérisée en ce que** le vernis protecteur est un vernis polyamide-imide ou un vernis en poudre.

16. Boîte de gaz propulseur selon la revendication 14 ou 15, **caractérisée en ce que** le clapet dans la tête de pulvérisation est un clapet à bille Ariane.
